# EUROPEAN PATENT APPLICATION

(11) **EP 4 194 012 A2**
(43) Date of publication of application: **14.06.2023**
(21) Application number: 22194047.1
(22) Date of filing: 06.09.2022
(51) Int. Cl.: A61L 2/00, A01M 13/00, A61L 2/20, A61D 7/00, A61M 13/00

(54) **PORTABLE PET AUDITORY MEATUS CARE DEVICE FOR STERILIZING AND REMOVING EAR MITES THROUGH OZONE**

(30) Priority: 07.12.2021 CN 202123053442 U
(71) Applicant: Shenzhen Heyi Precision Pump Tech Co., Ltd., Shenzhen, Guangdong 518100 (CN)
(72) Inventor: HU, Jun, Shenzhen, 518100 (CN); GE, Shaolong, Shenzhen, 518100 (CN); LI, Chunsheng, Shenzhen, 518100 (CN)
(74) Representative: Cabinet Chaillot

(57) **Abstract**

The present disclosure discloses a portable pet auditory meatus care device for sterilizing and removing ear mites through ozone. The device comprises a handle-shaped housing case, a nozzle for abutting against the auditory meatus which is provided with a connection port, an ozone generator for generating ozone gas which is connected with the output port of the nozzle, a drive board and a battery; when the nozzle is abutted against the opening of the auditory meatus, the output port is communicated with the interior of the auditory meatus. The device can completely disinfect the auditory meatus of pets so as to prevent and treat ear mites.

## Description

### TECHNICAL FIELD OF THE INVENTION

The present disclosure relates to a pet care equipment. In particular, the present disclosure relates to a portable pet auditory meatus care instrument for sterilizing and removing ear mites through ozone.

### BACKGROUND OF THE INVENTION

In prior art, the equipment used for sterilization and mite removal is generally related to ultraviolet light and drug spraying. Even though devices using ozone sterilization and disinfection have been disclosed, these devices are all electrical or medical devices with a relatively large volume. The existing equipment has many defects, such as large volume, high cost, inconvenient to carry, difficult to operate and so on.

At present, it is not disclosed the device which cleans and nurses pet auditory meatus using ozone to sterilize and remove ear mites. Especially the cleaning and nursing equipment as for various diseases of the auditory meatus of cats and dogs.

Many pet keepers think that the dirt in the pet's ears is caused by ear mites usually, but sometimes it may also be caused by Malassezia. Malassezia is a yeast, a single-celled fungus. It is a normal symbiotic microorganism on the skin of cats and dogs. Malassezia is usually parasitic in small amounts in the skin and mucous membrane junctions, such as lips, anus, vagina, foreskin, interdigital skin and the auditory meatus of cats and dogs. Malassezia pachydermatis (M. pachydermatis) is the main species parasitic on the skin of dogs. The most common species in cats is Malassezia pachyderm, but lipid-dependent yeast species may also be present, including Malassezia sympodialis (M. sympodialis), Malassezia furfur (M. furfur), etc... Under normal circumstances, Malassezia does not cause disease, but due to the promotion of certain factors, it can cause disease by multiplying and breaking down lipids and resulting in keratinocyte morphological changes and apoptosis. Conditions suitable for the growth of Malassezia include humidity, high temperature, abnormal keratinization, and an inflammatory environment, so any disruption of the skin microenvironment or immune defenses can lead to Malassezia overgrowth and infection and trigger Malassezia dermatitis. Microorganisms that cause Malassezia dermatitis and staphylococcal pyoderma may develop a symbiotic relationship due to reciprocal growth conditions, and the two diseases often occur simultaneously. Dogs with atopic dermatitis may have a hypersensitivity reaction to Malassezia, that is an abnormally high immune response, even a small number of Malassezia can cause severe itch in the pet and skin inflammation. M. pachydermatis may temporarily multiply on human skin. People can also be infected if they are immunocompromised. In clinical practice, the most direct method for diagnosing Malassezia is microscopic examination. Malassezia is oval or round or cylindrical or similar in shape to "peanut kernel" under microscope observation. Treatment methods include: 1) the use of antifungal drugs: according to the lesion site, appropriating ear medicines or topical medicines for skin diseases should be used; 2) regular cleaning of the lesions in cats and dogs: such as daily cleaning of the secretions in the auditory meatus of cats and dogs.

Ear mites, also known as sarcoptes scabiei or otodectes cynotis, are parasites commonly found in the auditory meatus of cats and dogs. Ear mites have a short life cycle of 18-28 days. The eggs of ear mites become larvae after 4 days. The larvae become adult worms after passing through the stages of nymphs and nymphs. Adult worms can fertilize and lay eggs. And the adult ear mites have strong vitality and can survive for 5 to 17 days after leaving the host. So, ear mites are highly contagious between cats and dogs. There can be thousands of ear mites in a cat's auditory meatus. Ear mites in dog auditory meatus are relatively small. Once cats and dogs are infected with ear mites, it is extremely difficult to completely cure. Nowadays, with the improvement of living environment and the improvement of civilization, pets have gradually become the important member of many families. The common pets include mostly cats and dogs due to the history of domestication and the degree of domestication. The characteristics of the physiological structure of the auditory meatus of cats and dogs make the atmosphere is high humidity, insufficient light, and poor ventilation in the auditory meatus. Then, the atmosphere provides suitable breeding conditions for ear mites. Ear mites cause unpleasant odors, itch, and severe cases, even cause inflammation and lead to abscess and ulcers. Many pet owners without keeping experience think that pets are smelly, which may lead to over-treatment or abandonment of pets.

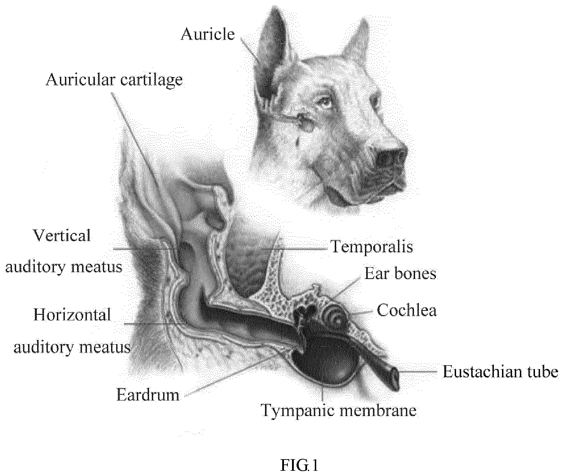

The most common way to treat pet ear mites is medication. Most of the medicines commercially available for treating ear mites are external medicines, including Acarexx containing ivermectin, Milbemite containing moxidectin, Tresaderm containing thiabendazole, Revolution containing selamectin, and Advocate containing moxidectin. A Chinese patent has disclosed a pharmaceutical composition for treating ear mites. The pharmaceutical composition is a transparent ear drops, containing the raw materials in weight percentage: 0.1%-1.0% of milbeoxime, 19.0%-92.5% of solvent, 2.0%-50.0% of solubilizer, and 2.0%-30.0% of stabilizer. The pharmaceutical composition can be dripped into the auditory meatus of cats and dogs, and has a direct effect on ear mites, and has a strong curative effect on moderate and severe infection by ear mites and be safety. But, as shown in FIG.1, the auditory meatus of cats and dogs comprises the vertical auditory meatus and the horizontal auditory meatus. The vertical auditory meatus is connected to the auricle, and the horizontal auditory meatus is connected to the tympanic membrane, eardrum, ear bones, cochlea, and eustachian tube. The angle between the vertical auditory meatus and the horizontal auditory meatus is close to 90°. After the treatment drug is instilled into the auditory meatus, due to its own tension, it only stays in the vertical auditory meatus, and cannot enter the horizontal auditory meatus through the 90° angle, so the effect of treating ear mites is greatly reduced, and the treatment time is long and incomplete. Even with a cotton swab as an aid, the swab cannot be bent and inserted into the horizontal auditory meatus.

### SUMMARY OF THE INVENTION

To solve the above-mentioned deficiencies in prior art, the present disclosure provides a portable pet auditory meatus care device that uses ozone to sterilize and remove mites. The device can completely disinfect the auditory meatus of cats and dogs and other pets and has a positive preventive and therapeutic effect on removing Malassezia and ear mites from the auditory meatus of pets.

In the present disclosure, the technical problems are solved through the following technical solutions.

A portable pet auditory meatus care device for sterilizing and removing ear mites through ozone, in which the device comprises:
a handle-shaped housing case, one end of which is provided with a connection port;
a nozzle having an output port, which is connected with the connection port for abutting against the auditory meatus, and the output port is communicated with the interior of the auditory meatus when the nozzle is abutted against the opening of the auditory meatus;
an ozone generator generating ozone gas, which is communicated with the output port of the nozzle;
a drive board for driving the ozone generator to generate ozone gas which is electrically connected with the ozone generator; and
a battery for providing power supply for the drive board which is electrically connected with the drive board; and
the ozone generator, the drive board and the battery are all disposed in the housing case.

In some examples, the battery is connected with the ozone generator by a switch, and the housing case is provided with a button corresponding to the switch for controlling the switch to be turned on or off.

In some examples, the nozzle is balloon-shaped and made of silica gel.

In some examples, the nozzle is connected with the housing case in a detachable manner.

In some examples, the axis of the housing case has an included angle of 1-90° relative to the axis of the nozzle.

In some examples, the device also comprises a power unit disposed in the housing case and communicating with the ozone generator for delivering the ozone gas generated by the ozone generator to the nozzle.

In some examples, the battery is connected with the power unit through the switch.

In some examples, the power unit is a micro fan, a micro fan blade, a micro air pump or a piezoelectric ceramic sheet.

In some examples, an air collecting cavity is formed between the nozzle and the power unit, in which the ozone generator is disposed; and
the air collecting cavity has a diameter which is gradually decreased from the first connection port connected with the power unit to the second connection port connected with the nozzle.

In some examples, the power unit is integrally connected with the ozone generator, the device further comprises an inner shell disposed in the housing case, which comprises:
a first cavity in which the power unit is disposed;
a second cavity in which the ozone generator is disposed;
an intake disposed between the first cavity and the inner cavity of the housing case, which is provided with a first one-way valve for passing air from the first cavity to the second cavity only;
an exhaust which is disposed between the second cavity and the output port of the nozzle; and
an interface disposed between the first cavity and the second cavity, which is provided with a second one-way valve for passing air from the first cavity to the second cavity only.

The device generates ozone by means of ozone generator, and releases ozone into the auditory meatus of cats or dogs and other pets through the output port on the nozzle. In this way, a complete disinfection of the auditory meatus is achieved. Ozone can diffuse to all corners of the complex auditory meatus of pets due to the indeterminate form and high fluidity. Even in the auditory meatus with a corner close to 90°, ozone can also enter through the corner, then enter the horizontal auditory meatus behind the corner to completely disinfect. The portable pet auditory meatus care device greatly improves the effect of treating ear mites, and shortens the treatment time, and improves the treatment efficiency, and has no drug residues in the pets' auditory meatus after treatment with high security.

### BRIEF DESCRIPTION OF THE DRAWINGS

FIG. 1 shows the schematic diagram of the dog's auditory meatus structure;
FIG.2 shows the internal schematic diagram of the portable pet auditory meatus care device according to the present disclosure;
FIG.3 shows the connection relationship of each component in the portable pet auditory meatus care device according to the present disclosure;
FIG.4 shows the schematic structural diagram of the portable pet auditory meatus care device according to the present disclosure when the power unit and the ozone generator are integrally connected; and
FIG.5 shows another schematic structural diagram of the portable pet auditory meatus care device according to the present disclosure when the power unit and the ozone generator are integrally connected.

### DETAILED DESCRIPTION OF THE INVENTION

The present disclosure will be described in detail below with reference to the accompanying drawings and embodiments, examples of which are shown in the accompanying drawings.

In the context, the same or similar reference numerals represent the same or similar elements or elements having the same or similar functions throughout. The embodiments described below with reference to the accompanying drawings are exemplary and are intended to explain the present disclosure and should not be construed as a limitation to the present disclosure.

It is to be understood that in this disclosure, the terms, such as "length", "width", "upper", "lower", "front", "rear", "left", "right", "vertical", "horizontal", "top", "bottom", "inside", "outer" and other indicated orientations or positional relationships, are based on the orientations or positional relationships shown in the accompanying drawings. And the terms are only for the convenience of describing the disclosure and simplifying the description, rather than indicating or implying that the device or element referred to must have a particular orientation or be constructed and operated in a particular orientation, so that the terms should not be construed as a limitation of the present disclosure. The terms are used only for convenience in describing the present disclosure and for simplifying the description, rather than indicating or implying that the referred device or element must have the particular orientation, and the particular orientation configuration and operation. Therefore, the terminology should not be construed as a limitation to the present disclosure.

Furthermore, terms such as "first", "second", and "third" are used for descriptive only and should not be construed as indicating or implying relative importance, nor the number of technical features. So, a feature defined as "first", "second", "third" may expressly or imply that the present disclosure comprises one or more of such features. In the description of the present disclosure, the meaning of "plurality" is two or more, unless explicitly defined.

In the present disclosure, unless expressly stated and defined, terms, such as "installed", "connected", "connected", "fixed", "arranged", should be construed broadly.

It can be understood that the elements may be fixedly connected, detachably connected, or integrally connected; may be mechanically connected or electrically connected; may be directly connected or indirectly connected through an intermediate medium. It can also be understood as a communication within two elements or an interaction relationship between two elements. For those skilled in the art, the specific meanings of the above terms in the present disclosure can be understood according to specific situations.

### The first embodiment

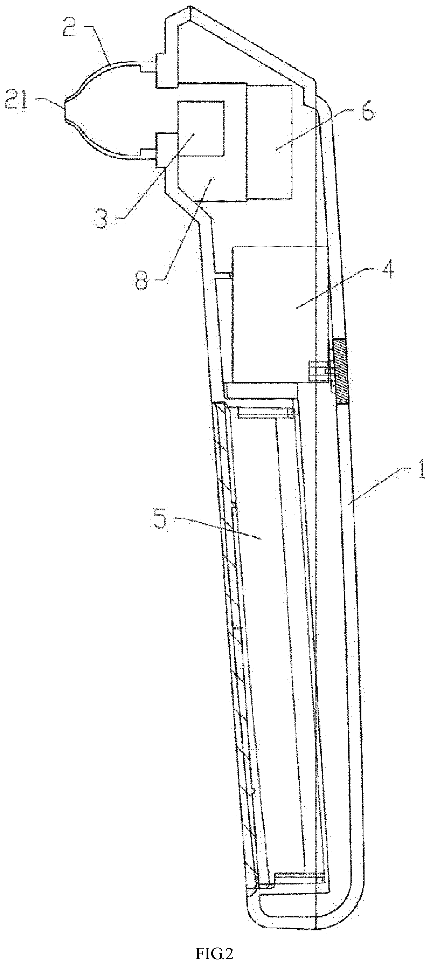

As shown in FIG.2, a portable pet auditory meatus care device for sterilizing and removing ear mites through ozone is shown. The device comprises a handle-shaped housing case 1, one end of which is provided with a connection port 11;
a nozzle 2 having an output port 21, which is connected with the connection port 11 for abutting against the auditory meatus, and the output port 21 is communicated with the interior of the auditory meatus when the nozzle 2 is abutted against the opening of the auditory meatus;
an ozone generator 3 for generating ozone gas, which is communicated with the output port 21 of the nozzle 2;
a drive board 4 for driving the ozone generator 3 to generate ozone gas, which is electrically connected with the ozone generator 3; and
a battery 5 for providing power supply for the drive board 4, and which is electrically connected with the drive board 4; and
the ozone generator 3, the drive board 4 and the battery 5 are all disposed in the housing case 1.

Ozone (O3) is a gas with strong oxidizing properties. Ozone has a fast, efficient, and comprehensive disinfecting effect on bacteria, viruses, fungi and even small parasites and mites. Ozone also has a better disinfecting effect in a high humidity environment and can remove odors. The treatments are physiotherapy and hormone-free, and the ozone is eventually converted to oxygen, leaving no residue.

The portable pet auditory meatus care device is obtained based on the strong oxidizing properties of ozone. The device generates ozone through the ozone generator 3, and releases ozone into the auditory meatus of cats, dogs, and other pets by the output port 21 on the nozzle. In this way, a complete disinfection of the auditory meatus is achieved. Because ozone gas is in a non-fixed form and has high mobility, after entering the auditory meatus of pets, ozone can diffuse to all corners of the auditory meatus for complete sterilization. Even in the auditory meatus structure with vertical and horizontal auditory meatus, such as cats and dogs, ozone can still pass from the vertical auditory meatus into the horizontal auditory meatus, through turning a 90° angle, then disinfect the horizontal auditory meatus. The portable pet auditory meatus care device greatly improves the effect on treating ear mites, and shortens the treatment time, and improves the treatment efficiency, and has no drug residues in the pets' auditory meatus after treatment, so the security is high.

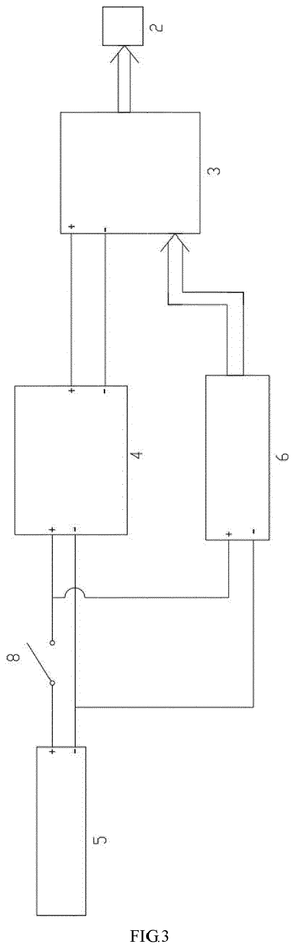

As shown in FIG.3, the battery 5 is connected with the ozone generator 3 by a switch, and the housing case 1 is provided with a button corresponding to the switch for controlling the switch to be turned on or off. Specifically, the positive pole of the battery 5 is electrically connected with the input positive pole of the drive board 4 through a switch, the negative pole of the battery 5 is directly electrically connected with the input negative pole of the drive board 4; and the output positive pole of the drive board 4 is electrically connected with the positive electrode of the ozone generator 3, and the output negative electrode of the drive board 4 is electrically connected with the negative electrode of the ozone generator 3.

Another switch button corresponding to the switch between the battery 5 and the drive board 4, is also disposed in the housing case 1, for controlling the switch between the battery 5 and the drive board 4 to be turned on or off.

The nozzle is balloon-shaped and made of silica gel. The material of the nozzle is soft, when it contacts with the pet's auditory meatus, it seems that the keeper is massaging the pet's auditory meatus, which can reduce the pet's discomfort. And preferably, the nozzle 2 is connected with the housing case 1 in a detachable manner.

Preferably, the nozzle 2 is snap-connected with the housing case 1.

Preferably, the axis of the housing case has an included angle of 1-90° relative to the axis of the nozzle 2.

The housing case is shaped like a handle. Ergonomic housing case 1 can be easily operated with one hand by the user's left and right hands.

In some embodiments, the battery 5 consists of two AA batteries. In other embodiments, the battery 5 is a lithium battery. Preferably, the battery is a rechargeable battery. The housing case 1 is further provided with a charging port, and the charging port electrically is connected with the battery 5 for charging the battery 5.

In the present embodiment, to speed up the output efficiency of ozone gas, the ozone generator 3 is disposed in the connection port 11 of the housing case 1. If there is no power unit 6, the ozone gas generated by the ozone generator 3 automatically passes into the auditory meatus after filling the entire compartment of the nozzle 2.

Of course, in other embodiments, the ozone generator 3 may be also disposed away from the nozzle 2. Then a hose or channel is disposed for communicating the ozone generator 3 with the nozzle 2.

### The second embodiment

As an improvement of the above-mentioned embodiment, as shown in FIG.2, the portable pet auditory meatus care device in this embodiment further comprises a power unit 6 disposed in the housing case 1 and communicating with the ozone generator 3 for delivering the ozone gas generated by the ozone generator 3 to the nozzle 2.

High concentrations of ozone are toxic. The portable pet auditory meatus care device transmits the ozone gas generated by the ozone generator 3 to the nozzle 2 through the power unit 6, which not only speeds up the speed of the ozone gas into the auditory meatus, but also shortens the treatment time. In addition, the air flow of the ozone injected into the auditory meatus is controlled by the power unit 6, which achieves the purpose of controlling the concentration of ozone gas in the auditory meatus and avoids the high concentration of ozone gas in the auditory meatus from causing damage to cats, dogs, and other pets, and improves safety.

To meet the acaricidal concentration of less than 5 cubic centimeters in the auditory meatus of cats and dogs, the power unit 6 controls the ozone injected into the auditory meatus to be maintained at about 0.02 mL, and the injection time is about 15 seconds during each treatment. Using the portable pet auditory meatus care device twice a week in daily life can not only prevent the breeding of ear mites, but also remove the odor of the auditory meatus; and in humid seasons, pets have a high incidence of ear mites, and the frequency of use of the device can be appropriately increased; as for pets already suffering from ear mites, combined with drug treatment will also shorten and improve the time and effect of treatment.

As shown in FIG.3, the battery 5 is connected with the power unit 6 by the switch.

Specifically, the positive electrode of the battery 5 is electrically connected with the positive electrode of the power unit 6 through the switch, and the negative electrode of the battery 5 is directly electrically connected with the negative electrode of the power unit 6. So that the switch can control the power supply of the battery 5 to the drive board 4 and the power unit 6 at the same time. The air outlet of the power unit 6 is communicated with the ozone generator 3 through an air passage.

The power unit 6 is preferably, but not limited to, a micro fan or a micro air pump.

An air collecting cavity 8 is formed between the nozzle 2 and the power unit 6, in which the ozone generator 3 is disposed; and
the air collecting cavity 8 has a diameter which is gradually decreased from the first connection port connected with the power unit 6 to the second connection port connected with the nozzle 2.

In this embodiment, the power unit 6 and the ozone generator 3 adopt a separate structure.

### The third embodiment

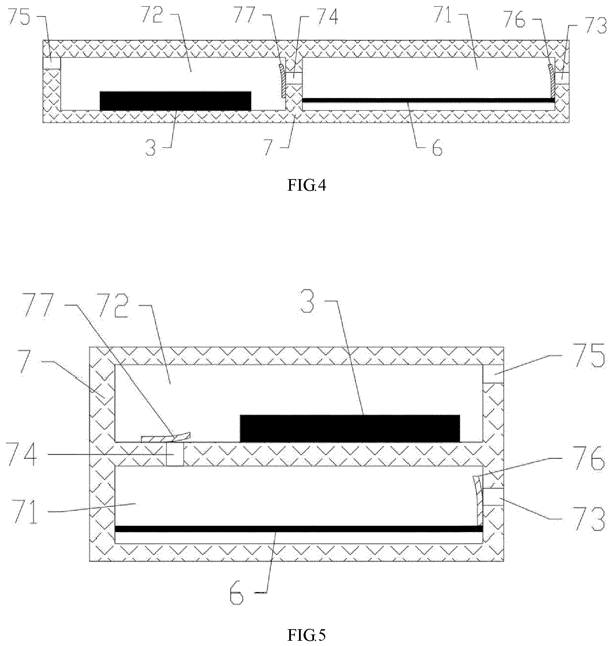

This embodiment is an improvement of the above-mentioned second embodiment. As shown in FIG.4 and FIG.5, the power unit 6 is integrally connected with the ozone generator 3. The device further comprises an inner shell 7 disposed in the housing case 1, which comprises:
a first cavity 71 in which the power unit 6 is disposed;
a second cavity 72 in which the ozone generator is disposed;
an intake 73 disposed between the first cavity 71 and the inner cavity of the housing case 1, which is provided with a first one-way valve 76 for passing air from the first cavity 71 to the second cavity 72 only;
an exhaust 73 which is disposed between the second cavity 72 and the output port 21 of the nozzle 2; and
an interface 74 disposed between the first cavity 71 and the second cavity 72, which is provided with a second one-way valve 77 for passing from the first cavity 71 to the second cavity 72 only.

The first cavity 71 and the second cavity 72 can be disposed side by side or can be stacked up and down.

In this embodiment, the power unit 6 is, but not limited to, a piezoelectric ceramic sheet or a miniature fan blade. The ozone generator 3 is an ozone ceramic sheet.

Finally, it should be noted that the above embodiments are only for illustrating the technical solutions of the present disclosure rather than limiting them. Although the embodiments of the present disclosure have been described in detail with reference to the preferred embodiments, those of ordinary skill in the art should understand that the technical solutions of the present disclosure can still be modified or equivalently replaced without departing from the scope of the technical solutions of the present disclosure, and these modifications or equivalent replacements should fall into the scope of present invention.

## Claims

1. A portable pet auditory meatus care device for sterilizing and removing ear mites through ozone, **characterized in that** the device comprises:
a handle-shaped housing case, one end of which is provided with a connection port;
a nozzle having an output port, which is connected with the connection port for abutting against the auditory meatus, and the output port is communicated with the interior of the auditory meatus when the nozzle is abutted against the opening of the auditory meatus;
an ozone generator for generating ozone gas which is communicated with the output port of the nozzle;
a drive board for driving the ozone generator to generate ozone gas which is electrically connected with the ozone generator; and
a battery for providing power supply for the drive board which is electrically connected with the drive board;
the ozone generator, the drive board and the battery are all disposed in the housing case.

2. The portable pet auditory meatus care device of claim 1, **characterized in that** the battery is connected to the ozone generator by a switch, and the housing case is provided with a button corresponding to the switch for controlling the switch to be turned on or off.

3. The portable pet auditory meatus care device of claim 1, **characterized in that** the nozzle is balloon-shaped and made of silica gel.

4. The portable pet auditory meatus care device of claim 1, **characterized in that** the nozzle is connected with the housing case in a detachable manner.

5. The portable pet auditory meatus care device of claim 1, **characterized in that** the axis of the housing case has an included angle of 1-90° relative to the axis of the nozzle.

6. The portable pet auditory meatus care device of claim 1, **characterized in that** the device also comprises a power unit disposed in the housing case and communicating with the ozone generator for delivering the ozone gas generated by the ozone generator to the nozzle.

7. The portable pet auditory meatus care device of claim 6, **characterized in that** the battery is connected with the power unit through a switch.

8. The portable pet auditory meatus care device of claim 6, **characterized in that** the power unit is a micro fan, a micro fan blade, a micro air pump or a piezoelectric ceramic sheet.

9. The portable pet auditory meatus care device of claim 6, **characterized in that** an air collecting cavity is formed between the nozzle and the power unit, in which the ozone generator is disposed; and
the air collecting cavity has a diameter which is gradually decreased from the first connection port connected with the power unit to the second connection port connected with the nozzle.

10. The portable pet auditory meatus care device of claim 6, **characterized in that** the power unit is integrally connected with the ozone generator, the device further comprises an inner shell disposed in the housing case, which comprises:
a first cavity in which the power unit is disposed;
a second cavity in which the ozone generator is disposed;
an intake disposed between the first cavity and the inner cavity of the housing case, which is provided with a first one-way valve for passing air from the first cavity to the second cavity only;
an exhaust which is disposed between the second cavity and the output port of the nozzle; and
an interface disposed between the first cavity and the second cavity, which is provided with a second one-way valve for passing air from the first cavity to the second cavity only.
